# EUROPEAN PATENT SPECIFICATION

(11) **EP 3 358 018 B1**
(45) Date of publication and mention of the grant of the patent: **18.09.2019**
(21) Application number: 17000189.5
(22) Date of filing: 07.02.2017
(51) Int. Cl.: C12N 9/12

(54) **METHOD FOR PREPARING A SOLUBLE AND FUNCTIONAL TELOMERASE COMPLEX IN EUKARYOTIC CELL-FREE SYSTEMS**
VERFAHREN ZUR HERSTELLUNG EINES LÖSLICHEN UND FUNKTIONELLEN TELOMERASEKOMPLEXES IN SYSTEMEN OHNE EUKARYOTISCHE ZELLEN
PROCÉDÉ DE PRÉPARATION D'UN COMPLEXE TÉLOMÉRASE FONCTIONNEL ET SOLUBLE DANS DES SYSTÈMES EUCARYOTES ACELLULAIRES

(43) Date of publication of application: 08.08.2018
(73) Proprietor: Fraunhofer-Gesellschaft zur Förderung der angewandten Forschung e.V., 80686 München (DE)
(72) Inventor: VON BRIESEN, Hagen, 65510 Hünstetten (DE); WAGNER, Sylvia, 66539 Neunkirchen (DE); STAB, Julia, 33189 Schlangen (DE); KUBICK, Stefan, 14109 Berlin (DE); ZEMELLA, Anne, 14469 Potsdam (DE); WÜSTENHAGEN, Doreen, 14478 Potsdam (DE)
(74) Representative: v. Bezold & Partner Patentanwälte - PartG mbB

(56) References cited:
- EP-A1- 1 686 186
- WO-A1-01/57227
- WO-A2-2015/043729
- GHADESSY FARID J ET AL: "A novel emulsion mixture for in vitro compartmentalization of transcription and translation in the rabbit reticulocyte system", PROTEIN ENGINEERING, DESIGN AND SELECTION, OXFORD JOURNAL, LONDON, GB, vol. 17, no. 3, 1 March 2004 (2004-03-01), pages 201-204, XP002485452, ISSN: 1741-0126, DOI: 10.1093/PROTEIN/GZH025
- TRAPP-FRAGNET LAETITIA ET AL: "The human telomerase catalytic subunit and viral telomerase RNA reconstitute a functional telomerase complex in a cell-free system, but not in human cells", CELLULAR & MOLECULAR BIOLOGY LETTERS, UNIVERSITY OF WROCAW. INSTITUTE OF BIOCHEMISTRY, WROCAW; PL, PL, vol. 17, no. 4, 1 September 2012 (2012-09-01), pages 598-615, XP035994718, ISSN: 1425-8153, DOI: 10.2478/S11658-012-0031-6 [retrieved on 2012-09-01]

## Description

### Background of the invention

A telomerase, such as human telomerase (hTERT), is a specialized reverse transcriptase that synthesizes telomeric DNA repeats to the ends of chromosomes. Telomeres are shortened during cell division based on a semi-conservative replication that leads to a loss of genetic information and aging of cells. Telomerase prevents shortening of telomeres by a still poorly understood procedure. For catalytic activity, the ribonucleoprotein complex requires two key subunits: telomerase reverse transcriptase subunit (in particular hTERT) and an intrinsic RNA (hTR) that serves as template for adding multiple G-rich repeats to the 3'end of telomeres. Due to the repeated usage of telomerase specific RNA as template, the nucleic acid is not degraded after transcription. This enzymatic mode of action significantly differs from viral reverse transcriptases. Moreover several DNA-binding proteins such as TRF1, TRF2, POT1, TIN2, Rap1 and TPP1 form a complex known as shelterin complex to protect telomere ends. Telomerase complex assembly is completed by participation of further factors such as chaperones (e.g. Hsp90, Hsp23) and dyskerin, an hTR binding and stabilizing protein. In somatic cells telomerase activity is usually undetectable whereas in nearly all cancer cells increased enzymatic activity was measured. In stem cells telomerase activity was also found. Due to the ability to prolong lifespan in both cell types, a correlation with telomerase activity is assumed.

For clinical trials it is important to obtain more information about the structure and mechanism of action of telomerase to develop new and effective drugs. Therefore a highly pure and active telomerase has to be synthesized. During the last years a huge number of *in vivo* experiments failed in the production of reasonable amounts of active full-length human telomerase (e.g. Mikuni et al. in Biochemical and Biophysical Research Communications 298, 144-150 (2002)). Typically, most of the synthesized hTERT was insoluble, probably due to incorrect folding and missing key components (such as dyskerin) which usually stabilize the telomerase complex.

Consequently, alternative approaches, in particular involving an *in vitro* synthesis of telomerase using various cell lysates, were pursued as well.

In recent years cell-free protein synthesis has evolved into a sophisticated method for protein production. Several translation systems were developed from both prokaryotic organisms (in particular *E. coli*) and eukaryotic organisms such as rabbit reticulocyte lysates, wheat germ lysates and insect lysates. Prokaryotic cell lysates, such as derived from *Escherichia coli,* usually generate high yields of proteins in a cost efficient manner, but only restricted posttranslational modifications (PTM) are possible in these systems due to missing membrane organelles.

Weinrich et al. (Nat. Genet. 17 (4), 498-502 (1997)) reported the cell-free synthesis of an active telomerase enzyme using a rabbit reticulocyte lysate and demonstrated that *in vitro* synthesis of telomerase in this system simply required the key units hTERT and hTR. However, the yields of active protein obtained with this system were rather low.

Some years later, Steigerwald et al. (Cell-free Translation Systems, 164-173 (2002)) achieved the synthesis of human telomerase in a cell-free system based on an *E. coli* lysate. This system, however, required the complementation by auxiliary factors, in particular folding-promoters such as Hsp70 and Hsp40, to obtain telomerase activity. These factors were separately synthesized and added to the translation mix. Further, the required telomerase specific RNA (hTR) was also separately synthesized by means of an *in vitro* transcription reaction, purified and - after completion of the cell-free translation reaction - added to the telomerase protein subunit and incubated once more. Thus, this approach is time-consuming and involves a rather large number of separate synthetic steps. Moreover, the obtained protein yields are still not quite satisfactory.

Ghadessy and Hollinger (Protein Engineering, Design and Selection, Vol. 17, No. 3, pp 201-204 (2004) developed an emulsion formulation for *in vitro* compartmentalisation of transcription and translation in the rabbit reticulocyte system which allowed *in-emulsion* expression of active human telomerase, in particular for screening purposes.

In view of the drawbacks of the prior art, the main object of the present invention was to provide improved means for preparing a soluble and functional telomerase complex, in particular a human telomerase complex, which allows to obtain the desired complex in a fast and efficient manner and in high yields as well.

This objective has been achieved in principle by providing the method according to present claim 1. More specific and/or preferred embodiments of the invention are the subject of further claims.

### Description of the invention

The present invention is based on the surprising finding that it is not only possible to synthesize the telomerase protein subunit (TERT) and the RNA subunit (TR) concomitantly *in vitro* in the same reaction medium containing an eukaryotic cell lysate but also to obtain a soluble and active telomerase complex by self-assembling of both components in said reaction medium without any purification or isolation steps. Thus, the method of the invention is considerably simplified as compared to the methods of the prior art. Moreover, as demonstrated in the experimental section below, the yields with respect to the amount of synthesized protein as well as to active telomerase complex are considerably higher than those obtained by any other method of the art.

The method for preparing a soluble and functional telomerase complex in cell-free systems according to claim 1 comprises at least the following steps:
a) providing a reaction medium comprising at least the following components
   - a nucleic acid template coding for the telomerase reverse transcriptase subunit (TERT)
   - a DNA template coding for the intrinsic telomerase RNA (TR),
   - an RNA polymerase
   - an eukaryotic cell lysate;
b) concomitantly performing a translation reaction to synthesize the telomerase reverse transcriptase subunit (TERT) and a transcription reaction to synthesize the intrinsic telomerase RNA (TR);
c) self-assembling of the TERT protein subunit and the TR RNA subunit in the reaction medium to form a functional telomerase complex;
d) optionally further purifying and/or isolating the telomerase complex,
characterised in that the method is carried out in a device which comprises at least two compartments separated by a dialysis membrane, wherein the translation and transcription reactions take place in at least one first compartment, the reaction compartment, and, during the translation and transcription reactions, i) reactants diffuse through the dialysis membrane out of at least one further compartment, the supply and discharge compartment, into the reaction compartment and ii) reaction by-products diffuse through the dialysis membrane out of the reaction compartment into the supply and discharge compartment.

The telomerase may be principally any telomerase, in particular any vertebrate or, more specifically, mammalian telomerase, and preferably is human telomerase.

The nucleic acid template coding for the telomerase reverse transcriptase subunit (TERT) may be DNA or RNA and may be provides in linear or circular form. A circular template such as incorporated into a plasmid, is generally preferred. Since the coding nucleic sequence of, e.g., the hTERT protein is known, a suitable expression construct comprising said template as well as suitable promoters and regulatory sequences can be readily designed as desired using common vectors and promoters. Some suitable but not limiting examples for such promoters are SP6, T3, and T7 promoters.

The choice of the RNA polymerase is not especially limited and depends on the specific promoters in the respective nucleic acid templates. For example, the RNA polymerase may be selected from the group comprising T3, T7 polymerase, and SP6 polymerase.

The eukaryotic cell lysate used in the method of the invention is preferably selected from the group comprising wheat germ lysates, insect cell lysates, cell extracts from CHO cells and human cells such as HEK cells.

More specifically, the insect cell lysates may be Spodoptera frugiperda cell lysates, in particular Sf21 or Sf9 cell lysates.

These cell lysates can be used in their native form or modified by addition or removal of particular components.

In one preferred embodiment of the invention, the cell lysate contains membrane vesicles.

Preferably, these membrane vesicles originate from the same cell line as the cell lysate. For example, insect cell lysates, cell extracts from CHO cells and human cells such as HEK cells contain such membrane vesicles (microsomes) in their native form.

However it is also possible to add membrane vesicles from other sources to a specific cell lysate, e.g. to a cell lysate which originally does not contain membrane vesicles.

The reaction medium comprises said cell lysate which contains the essential components for translation such as ribosomes, translation factors, enzymes. Furthermore, the medium or cell lysate is supplemented with amino acids, ATP and GTP and an energy regenerating system (e.g. creatine-phosphate/creatine-kinase energy-regenerating system). Synthesis of the target protein is initiated by the addition of an appropriate nucleic acid template for the target protein, either in form of DNA or mRNA.

Further the reaction medium comprises all the essential components for the transcription reaction as well, in particular a DNA template coding for the intrinsic telomerase RNA (TR), for example incorporated into a plasmid vector.

Cell-free protein synthesis reactions can be realised experimentally in a variety of ways. The simplest reaction route is the synthesis of a target protein in a one-pot synthesis (or batch reaction). Batch-based systems are therefore suitable for uncomplicated and rapid synthesis of a target protein. On the other hand, however, typically they are characterised by short run times and relatively low protein yields.

Therefore, advantageously the method according to the invention is performed continuously in a per se known dialysis system. Herein, i.a. energy-rich substances such as ATP and GTP and other pass by diffusion through a membrane into the reaction compartment, the site of the translation. At the same time, the reaction is depleted of inhibiting substances such as free phosphates and ADP. The continuous supply of the cell-free reaction in the reaction compartment prolongs the run time of the synthesis and leads to significantly raised protein yields as compared with the batch system.

The claimed method is realized in a device which comprises at least two compartments separated by a dialysis membrane, wherein the translation and transcription reactions take place in at least one first compartment, the reaction compartment, and, during the translation reaction, i) reactants diffuse through the dialysis membrane out of at least one further compartment, the supply and discharge compartment, into the reaction compartment and ii) reaction by-products diffuse through the dialysis membrane out of the reaction compartment into the supply and discharge compartment.

In a further preferred embodiment of the invention, the reaction medium further comprises a caspase inhibitor.

When a continuous dialysis system as outlined above is used, the caspase inhibitor is present at least in the reaction compartment, although it can preferably also be present in the supply and discharge compartment in order to supply further fresh inhibitor to the reaction compartment.

The presence of the caspase inhibitor in the reaction mixture, in particular in a continuous system, enables substantially longer run times for the reaction (for example, up to 48 h or even longer) and thereby leads to significant protein yield increases. The presence of a caspase inhibitor is particularly advantageous in systems involving insect cell lysates or CHO cells.

The caspase inhibitor may be any known general inhibitor of a caspase, in particular of one of the currently known caspase types 1-14. Suitable caspase inhibitors are described, for example, in the international patent application PCT/EP2014/002520. One non-limiting example thereof is the peptide derivative Z-VAD-FMK.

### Brief description of the Figures

**Fig. 1** shows the relative and absolute amounts of HTERT protein synthesized with a wheat germ lysate in absence of hRT, in the presence of separately transcribed and added hRT, and in the presence of parallel transcribed hTR (referred to as hTR(plasmid).
**Fig. 2** shows the relative telomerase activity of a sample from a wheat germ lysate based translation mix containing no hTR versus a sample from a reaction mix containing separately transcribed hTR and versus a sample from reaction mix containing parallel transcribed hTR (referred to as hTR(plasmid).

The following examples illustrate the present invention in more detail, however, without limiting the same to the specific parameters and conditions thereof.

### EXAMPLE 1

### Synthesis of a functional human telomerase complex using eukaryotic cell lysates

### General materials and methods

### 1. Plasmid constructs

hTERT encoding pMA-hTERT-His plasmid was obtained from GeneArt, Life Technologies. Plasmid for protein synthesis in wheat germ systems was obtained from PCR using suitable primers for cloning in the expression vector pIVEX1.3 WG. A plasmid containing the telomerase RNA component (hTR) nucleotide sequence (NCBI; Gene ID: 7012) was manufactured by GeneArt (Life Technologies GmbH, Darmstadt, Germany).

### 2. DNA template design

Linear DNA template for synthesis of hTERT in wheat germ system was generated according to the manufactures instructions RTS Wheat Germ LinTempGenSet, His₆-Tag Kit, biotechrabbit.

### 3. Preparation of eukaryotic cell lysates

*Sf21* cells were grown in fermenters at 27°C, CHO cells and human cell lines were grown at 37 °C in an animal component free medium (*Sf21*: Insect-XPRESS medium, Lonza; CHO: Power CHO-2 CD medium, Lonza; K562: ISF-1 serum free medium, InVivo) and harvested at a density of approximately 4 x 10⁶ cells/ml (Sf21) and 2 x 10⁶ cells/ml (CHO and K562) respectively. Cells were collected by centrifugation and washed with a HEPES-based [4-(2-hydroxyethyl)-1-piperazineethanesulfonic acid] buffer [40 mM HEPES-KOH (pH 7.5), 100 mM KOAc and 4 mM Dithiothreitol (DTT)]. Afterwards cell pellets were resuspended, disrupted mechanically and centrifuged at 10000 x g for 15 min at 4 °C to remove the nuclei and debris (Kubick et al. 2003). The supernatant was applied to a Sephadex G-25 column (GE Healthcare) and fractions with highest protein/ RNA concentrations were pooled. To degrade residual mRNA, fractions were treated with micrococcal nuclease (S7). Aliquots of the Sf lysate were immediately frozen in liquid nitrogen and stored at -80 °C to preserve maximum activity.

### 4. Eukaryotic cell-free protein synthesis

Protein synthesis based on lysates derived from cultured human and CHO cells was carried out in a linked transcription/translation mode. In this mode transcription was performed in a previous step by using T7 RNA-polymerase (Agilent, Orth et al. 2011). Generated mRNA was purified by an intermediate gel filtration step (DyeEx spin columns, Qiagen). Subsequently purified mRNA was added to the reaction mixture composed of 40 % (v/v) lysate (human or CHO), amino acids (200 µM) and energy generating components (1.75 mM ATP, 0.45 mM GTP). Translation reactions were performed in 25 µl batch volumes for 2 h at 33 °C (human) and 30 °C (CHO), respectively.

Cell-free protein synthesis based on insect extracts was performed in a previously described coupled transcription/ translation mode in 25 µl batch volumes at 27 °C for 2 h (Brödel et. al 2013) Reaction mixture was made of 40 % (v/v) lysate, amino acids (100 µM) and energy generating components (1,75 mM ATP, 0.45 mM GTP). ¹⁴C-leucine (46.15 dpm/ pmol) was added to reaction mixture to determine the size, integrity of the *de novo* synthesized protein of interest and yield.

Synthesis in wheat germ lysates was performed in a dialysis system using the RTS100 Wheat Germ CECF Kit (biotechrabbit) for 24 h at 24 °C according to the manufacturer's instructions. Again ¹⁴C-leucine (2.47 dpm/ pmol) was added to the reaction mixture to determine the size, integrity of the *de novo* synthesized protein of interest and yield.

### 5. Incorporation of the ncRNA component of human telomerase

RNA component of telomerase was incorporated in two different ways: RNA was generated in a previous transcription reaction by using T7 RNA - polymerase followed by a RNA purification using DyeEx spin columns (Qiagen) to add freshly synthesized RNA in a coupled and linked reaction, respectively. Alternatively, plasmid harboring the nucleotide sequence encoding the RNA component of human telomerase under control of the T7-promoter was added directly (coupled reaction) to the translation reaction.

### 6. Determination of protein yield

Protein yields were determined by hot trichloroacetic acid (TCA) precipitation followed by a radioactivity measurement. 5 µl aliquots of each translation reaction were mixed with 3 ml TCA and transferred in a water bath with a temperature of 80 °C for 15 min followed by an incubation time of 30 min on ice. Afterwards the mixtures were filtered using a vacuum filtration system (Hoefer) to collect the radiolabeled proteins on the surface of the filter paper (MN GF-3, Machery-Nagel). Filters were washed twice with TCA and acetone. Dried protein-enriched filters were transferred to scintillation tubes (Zinsser Analytic) covered with 3ml of scintillation cocktail (Quicksafe A, Zinsser Analytic) and left shaking using an orbital shaker (Unimax 1010, Heidolph) for at least 1 h. For measurement of incorporated ¹⁴C-leucine, liquid scintillation counting was performed using the LS6500 Multi-Purpose scintillation counter (Beckman Coulter).

### 7. SDS-PAGE

To verify homogeneity and molecular weight of synthesized proteins, 5 µl aliquots of radiolabeled translation mixture were subjected to cold acetone precipitation. Pellets were resuspended in 20 µl of 1 x sample buffer (NuPAGE® LDS Sample Buffer, Invitrogen) and electrophoretically separated on 10 % precast SDS-PAGE gels (NuPAGE® 10 % Bis-Tris Gel with MES SDS buffer, Invitrogen). SDS-PAGE gels were run at 200 V for 35 min. Subsequently gels were dried at 70 °C (Unigeldryer 3545D, Uniequip) and radioactively labeled proteins were visualized using a phosphorimager system (Typhoon TRIO + Imager, GE Healthcare).

### Results

Human telomerase was synthesized in cell-free systems based on wheat germ cell lysate as indicated in sections 4. and 5. above. In order to assess the influence of parallel transcribed hTR with respect to the translation reaction, analogous translation reactions were performed in absence of hRT, in the presence of separately transcribed and added hRT, and in the presence of parallel transcribed hTR (referred to as hTR(plasmid).

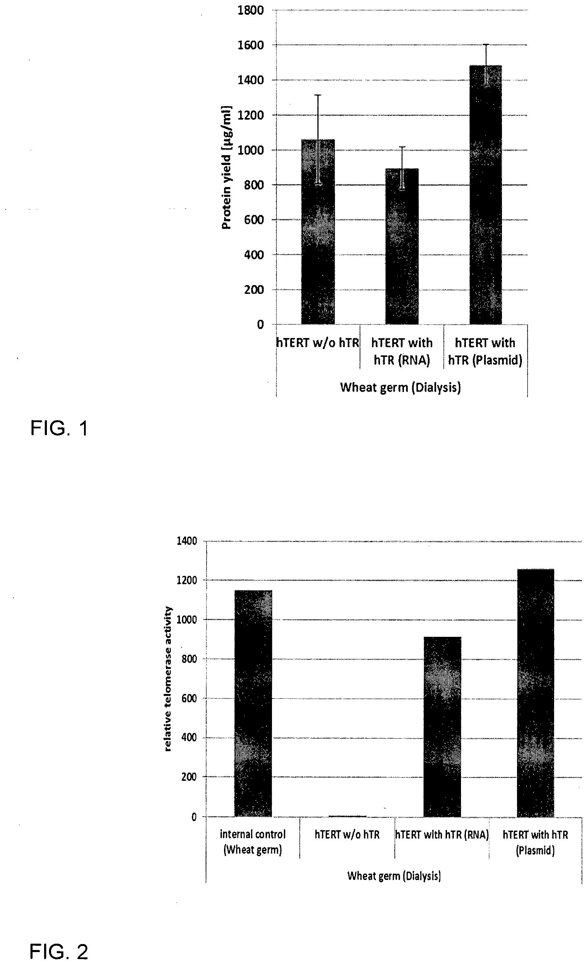

Fig. 1 shows the relative and absolute amounts of HTERT protein synthesized under these conditions. Whereas the addition of separately transcribed hTR resulted in similar protein yields as the translation reaction without the presence of any hTR (ca 900 µg/ml), the translation reaction in the presence of transcribed hRT plasmid surprisingly resulted in a considerable increase up to 1.5 mg/ml.

### EXAMPLE 2

### Functional analysis of telomerase using a TRAP assay

Functionality of telomerase was assessed by the telomeric repeat amplification protocol (TRAP-assay; Wright et al. 1995) using the TeloTAGGG Telomerase PCR ELISA^{PLUS} Kit (Roche). For functional analysis, 1 µl aliquot of the translation mixture was used for polymerase chain reaction (PCR) where active telomerase adds telomeric repeats (TTAGGG) to the 3'-end of a biotin-labeled primer. The resulting products containing the telomere specific repeats were amplified by PCR. In the next step a 2.5 µl aliquot of the PCR product was denatured and hybridized to a digoxigenin-(DIG) - labeled probe that recognizes telomeric repeats. The product consisting of telomeric repeats, the DIG-probe and the biotin-labeled primer were immobilized to a strepavidin-coated microplate and detected with a peroxidase conjugated antibody against digoxigenin (anti-DIG-POD). By adding tetramethylbenzidine (TMB) substrate a colored complex was formed displaying an absorbance which could be measured at 450 nm with a reference wavelength of 690 nm. The relationship between the absorption of an internal standard and the sample gives the relative telomerase activity.

In all systems the assay was performed in the same way by pipetting 1 µl of the rude translation mixture to the PCR mixture.

Fig. 2 shows the relative telomerase activity of a sample from a wheat germ lysate based translation mix containing no hTR versus a sample from a reaction mix containing separately transcribed hTR and versus a sample from a reaction mix containing parallel transcribed hTR (referred to as hTR(plasmid). As internal control a construct was added that imitates telomerase activity.

The telomerase activity obtained with the sample from a reaction mix containing parallel transcribed hTR was considerably higher than that of a sample from a corresponding reaction mix containing separately transcribed hTR. It appears that the functional telomerase complex is very efficiently self-assembled during the concomitant translation/transcription reactions.

## Claims

1. A method for preparing a soluble and functional telomerase complex in cell-free systems comprising at least the following steps:
a) providing a reaction medium comprising at least the following components:
- a nucleic acid template coding for the telomerase reverse transcriptase subunit (TERT)
- a DNA template coding for the intrinsic telomerase RNA (TR),
- an RNA polymerase
- an eukaryotic cell lysate;
b) concomitantly performing a translation reaction to synthesize the telomerase reverse transcriptase subunit (TERT) and a transcription reaction to synthesize the intrinsic telomerase RNA (TR);
c) self-assembling of the TERT protein subunit and the TR RNA subunit in the reaction medium to form a functional telomerase complex;
d) optionally further purifying and/or isolating the telomerase complex,
**characterised in that** the method is carried out in a device which comprises at least two compartments separated by a dialysis membrane, wherein the translation and transcription reactions take place in at least one first compartment, the reaction compartment, and, during the translation and transcription reactions, i) reactants diffuse through the dialysis membrane out of at least one further compartment, the supply and discharge compartment, into the reaction compartment and ii) reaction by-products diffuse through the dialysis membrane out of the reaction compartment into the supply and discharge compartment.

2. The method according to claim 1, wherein the telomerase is human telomerase.

3. The method according to claim 1 or 2, wherein the RNA Polymerase is selected from the group comprising T3, T7 polymerase, and SP6 Polymerase.

4. The method according to any one of claims 1-3, wherein the eukaryotic cell lysate is selected from the group comprising wheat germ lysates, insect cell lysates, cell extracts from CHO and HEK cells.

5. The method according to any one of claims 1-4, wherein the insect cell lysates are Spodoptera frugiperda cell lysates, in particular Sf21 or Sf9 cell lysates.

6. The method according to any one of claims 1-5, wherein the cell lysate contains membrane vesicles.

7. The method according to claim 6, **characterised in that** the membrane vesicles originate from the same cell line as the cell lysate.

8. The method according to any one of claims 1-7, wherein the template coding for the telomerase reverse transcriptase subunit (TERT) is linear or circular, preferably circular.

9. The method according to any one of claims 1-6, wherein the reaction medium further comprises a caspase inhibitor.

## Patentansprüche

1. Verfahren zur Herstellung eines löslichen und funktionellen Telomerase-Komplexes in zellfreien Systemen, umfassend mindestens die folgenden Schritte:
a) Bereitstellen eines Reaktionsmediums, welches mindestens die folgenden Komponenten umfasst:
- eine Nukleinsäure-Matrize, welche für die Reverse-Transkriptase-Untereinheit der Telomerase (TERT) codiert,
- eine DNA-Matrize, welche für die intrinsische Telomerase-RNA (TR) codiert,
- eine RNA Polymerase,
- ein eukaryotisches Zelllysat;
b) gleichzeitiges Durchführen einer Translationsreaktion, um die Reverse-Transkriptase-Untereinheit der Telomerase (TERT) zu synthetisieren, und einer Transkriptionsreaktion, um die intrinsische Telomerase-RNA (TR) zu synthetisieren;
c) Selbstassemblierung der TERT-Proteinuntereinheit und der TR-RNA-Untereinheit in dem Reaktionsmedium, um einen funktionellen Telomerase-Komplex zu bilden;
d) gegebenenfalls weiteres Reinigen und/oder Isolieren des Telomerase-Komplexes,
**dadurch gekennzeichnet, dass** das Verfahren in einer Vorrichtung durchgeführt wird, die mindestens zwei durch eine Dialysemembran getrennte Kompartimente umfasst, wobei die Translations- und Transkriptionsreaktionen in mindestens einem ersten Kompartiment, dem Reaktionskompartiment, stattfinden und während der Translations- und Transkriptionsreaktionen i) Reaktanden aus mindestens einem weiterem Kompartiment, dem Ver- und Entsorgungskompartiment, durch die Dialysemembran in das Reaktionskompartiment diffundieren und ii) Reaktionsnebenprodukte durch die Dialysemembran aus dem Reaktionskompartiment in das Ver- und Entsorgungskompartiment diffundieren.

2. Verfahren nach Anspruch 1, wobei die Telomerase humane Telomerase ist.

3. Verfahren nach Anspruch 1 oder 2, wobei die RNA-Polymerase aus der Gruppe ausgewählt ist, welche T3-, T7-Polymerase und SP6-Polymerase umfasst.

4. Verfahren nach irgendeinem der Ansprüche 1-3, wobei das eukaryotische Zelllysat aus der Gruppe ausgewählt ist, welche Weizenkeimlysate, Insektenzelllysate, Zellextrakte aus CHO- und HEK-Zellen umfasst.

5. Verfahren nach irgendeinem der Ansprüche 1-4, wobei die Insektenzelllysate *Spodoptera frugiperda*-Zelllysate, insbesondere *Sf*21- oder *Sf*9-Zelllysate, sind.

6. Verfahren nach irgendeinem der Ansprüche 1-5, wobei das Zelllysat Membranvesikel enthält.

7. Verfahren nach Anspruch 6, **dadurch gekennzeichnet, dass** die Membranvesikel von derselben Zelllinie wie das Zelllysat stammen.

8. Verfahren nach irgendeinem der Ansprüche 1-7, wobei die Matrize, welche für die Reverse-Transkriptase-Untereinheit der Telomerase (TERT) kodiert, linear oder zirkulär, vorzugsweise zirkulär, ist.

9. Verfahren nach irgendeinem der Ansprüche 1-6, wobei das Reaktionsmedium ferner einen Caspase-Inhibitor umfasst.

## Revendications

1. Procédé pour préparer un complexe de télomérase fonctionnel et soluble dans des systèmes acellulaires comprenant au moins les étapes suivantes :
a) la fourniture d'un milieu réactionnel comprenant au moins les composants suivants :
- une matrice d'acide nucléique codant pour la sous-unité de télomérase transcriptase inverse (TERT)
- une matrice d'ADN codant pour l'ARN télomérase intrinsèque (TR),
- une ARN polymérase
- un lysat de cellule eucaryote ;
b) la mise en oeuvre de manière concomitante d'une réaction de traduction pour synthétiser la sous-unité de télomérase transcriptase inverse (TERT) et d'une réaction de transcription pour synthétiser l'ARN télomérase intrinsèque (TR) ;
c) l'auto-assemblage de la sous-unité de protéine TERT et de la sous-unité d'ARN TR dans le milieu réactionnel pour former un complexe de télomérase fonctionnel ;
d) facultativement en outre la purification et/ou l'isolement du complexe de télomérase,
**caractérisé en ce que** le procédé est réalisé dans un dispositif qui comprend au moins deux compartiments séparés par une membrane de dialyse, dans lequel les réactions de traduction et de transcription ont lieu dans au moins un premier compartiment, le compartiment réactionnel, et, lors des réactions de traduction et de transcription, i) les réactifs se diffusent à travers la membrane de dialyse hors d'au moins un compartiment supplémentaire, le compartiment d'alimentation et d'évacuation, dans le compartiment réactionnel et ii) les sous-produits réactionnels se diffusent travers la membrane de dialyse hors du compartiment réactionnel dans le compartiment d'alimentation et d'évacuation.

2. Procédé selon la revendication 1, dans lequel la télomérase est une télomérase humaine.

3. Procédé selon la revendication 1 ou 2, dans lequel l'ARN polymérase est sélectionnée dans le groupe comprenant la polymérase T3, T7, et la polymérase SP6.

4. Procédé selon l'une quelconque des revendications 1 à 3, dans lequel le lysat de cellule eucaryote est sélectionné dans le groupe comprenant les lysats de germe de blé, les lysats de cellule d'insecte, les extraits cellulaires provenant de cellules CHO et HEK.

5. Procédé selon l'une quelconque des revendications 1 à 4, dans lequel les lysats de cellule d'insecte sont des lysats de cellule de Spodoptera frugiperda, en particulier des lysats de cellules Sf21 ou Sf9.

6. Procédé selon l'une quelconque des revendications 1 à 5, dans lequel le lysat de cellule contient des vésicules membranaires.

7. Procédé selon la revendication 6, **caractérisé en ce que** les vésicules membranaires proviennent de la même lignée cellulaire que le lysat de cellule.

8. Procédé selon l'une quelconque des revendications 1 à 7, dans lequel la matrice codant pour la sous-unité de télomérase transcriptase inverse (TERT) est linéaire ou circulaire, de préférence circulaire.

9. Procédé selon l'une quelconque des revendications 1 à 6, dans lequel le milieu réactionnel comprend en outre un inhibiteur de caspase.
